Europäisches Patentamt

European Patent Office

Office européen des brevets

(19)

(11) **EP 0 849 686 A2**

(12) **EUROPÄISCHE PATENTANMELDUNG**

(43) Veröffentlichungstag:
**24.06.1998 Patentblatt 1998/26**

(51) Int. Cl.$^6$: **G06F 17/00**, A61B 5/00

(21) Anmeldenummer: **97117332.3**

(22) Anmeldetag: **07.10.1997**

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **18.10.1996 DE 19643130**
**05.11.1996 DE 19645579**

(71) Anmelder:
**N.I.R. Control Medizintechnik GmbH**
**91456 Diespeck (DE)**

(72) Erfinder:
 • **Krauss, Manfred, Prof., Dr.-Ing. habil.**
  **09126 Chemnitz (DE)**
 • **Grohmann, Gerald, Dr. med.**
  **07743 Jena (DE)**
 • **Bilz, Dietrich, Dr. sc.med.**
  **12623 Berlin (DE)**

(74) Vertreter:
 **Kruspig, Volkmar, Dipl.-Ing. et al**
 **Patentanwälte**
 **Meissner, Bolte & Partner**
 **Postfach 86 06 24**
 **81633 München (DE)**

(54) **Vorrichtung und Arbeitsverfahren zum Betreiben der Vorrichtung zum Bestimmen und Auswerten von Funktionszuständen im Herz-Kreislauf-System**

(57)  Die Erfindung betrifft eine Vorrichtung und ein Arbeitsverfahren zum Betreiben der Vorrichtung zum Bestimmen und Auswerten von Funktionszuständen im Herz-Kreislauf-System. Erfindungsgemäß werden die Funktionszustände im Herz-Kreislauf-System, insbesondere der Blutgefäße einschließlich des Herzens im Zusammenwirken mit dem autonomen Nervensystem dadurch ermittelt, daß unter Rückgriff auf eine an sich bekannte Zwei-Wellenlängen-NIR-ROT-Remissions-Photoplethysmographie die Herzperiodendauer, das relative mikrovaskuläre Füllungsvolumen sowie der Oxygenierungsgrad des Blutes je Herzaktion erfaßt und entsprechende Zeitreihen-Tachogramme abgeleitet werden. Diese Zeitfunktionen werden einer Auto- und/oder Kreuzkorrelationsbildung unterzogen. Die erhaltenen Funktionen werden gespeichert, mit Normalwerten verglichen und/oder einer graphischen Darstellung unterzogen, so daß Aussagen beispielsweise über die Auswirkung von Pharmaka oder Drogen gewonnen werden können und/oder daß Größen ableitbar sind, die zur Patientenüberwachung herangezogen werden können.

EP 0 849 686 A2

**Beschreibung**

Die Erfindung betrifft eine Vorrichtung und ein Arbeitsverfahren zum Betreiben der Vorrichtung zum Bestimmen und Auswerten von Funktionszuständen im Herz-Kreislauf-System.

Aus Steinmann, J. et al: "In-vivo-Messung der Sauerstoffsättigung des Blutes mit Quarzlichtleitern und einem optischen Vielkanalanalysator", Biomed.Technik 31 (1986), 246 - 251 ist es bekannt, mittels NIR-Rot-Remissions-Photoplethysmographie wie folgt zu messen:

- Mit der Wellenlänge 840nm die relative Menge des Blutes bzw. der Erythrozyten im illuminierten Gewebe, unabhängig von dessen Oxygenierungsgrad, da bei Remissionsmessung in vivo der sog. $Hb/HbO_2$-isobestische Wellenlängenbereich bei 840 ... 850nm liegt. Als isobestisch wird dabei definitionsgemäß jener Wellenlängenbereich einer Strahlung bzw. des Lichts bezeichnet, bei dem unterschiedliche chemische Stoffe, z.B. Hämoglobin und Oxyhämoglobin, das durch diese Stoffe hindurchtretende Licht gleichermaßen schwächen, d.h. deren Extinktions- bzw. Schwächungskoeffizient gleich ist. Die NIR-Wellenlänge 840nm mißt somit relativ zum illuminierten Gesamtgewebe die Blut- bzw. Erythrozytenmenge, die die Mikrogefäße nach dem Fåhraeus-Effekt in Form von Blutvolumenpulswellen passiert.
- Mit der Wellenlänge 640nm andererseits die durch 840nm erfaßte relative Blut bzw. Erythrozytenmenge hinsichtlich ihres Oxygenierungsgrades, indem sich das meßbare Lichtintensitätsverhältnis zwischen 640nm und 840nm während der Mikrogefäß- bzw. Kapillarpassage infolge Desoxygenierung ändern. Aus der Intensitätsänderung des Rückstreusignals beider Wellenlängen läßt sich sowohl die allgemeine Änderung der Durchblutung des illuminierten Gewebes als auch die dazu relative Menge der die desoxygenierenden Kapillaren passierenden Erythrozyten quantitativ, d.h. oxymetrisch bestimmen, wie in herkömmlichen Oxymetern realisiert ist. Man definiert die Sauerstoffsättigung $SaO_2$ des arteriellen Blutes als das Verhältnis von sauerstoffangereichertem Hämoglobin ($HbO_2$) und Gesamthämaglobin ($HbO_2$ + Hb + sonstige Formen, wie Carbosyhämoglobin, Methämoglobin, Sulfhämoglobin):

$$SaO_2 = \frac{HbO_2}{HbO_2 + hb + Sonstige}$$

[nach Handbuch Ohmeda Biox 3740 Pulse]

Andererseits wird allgemein die Sauerstoffsättigung als Verhältnis von $O_2$-Gehalt (in Vol.-%) zu $O_2$-Kapazität (in Vol.-%) definiert (Netter, F.H.: Farbatlanten der Medizin, Bd. 1: Herz, 3.überarb.u.erw. Auflage, The Ciba Collection of Medical Illustrations, 1993):
Ein Normalwert von 97% Sättigungsgrad des Blutes im Kapillarbett wird erhalten bei einem $O_2$-Gehalt von 20,4 Vol.-% und einer $O_2$-Kapazität von 21 Vol.-%.
Ebenso wird eine arteriovenöse Differenz $avDO_2$ der $O_2$-Gehalte eingeführt (Schmidt, R.F.; Thews, G.: Physiologie des Menschen. 23. Aufl., Springer-Verlage Berlin/Heidelberg/New York/London/Paris/Tokyo 1987), indem mit den Werten für die arterielle $O_2$-Sättigung ($SaO_2$ = 97%) und die venöse $O_2$-Sättigung ($SaO_2$ = 73%) ein Gehalt an chemisch gebundenem Sauerstoff im arteriellen und venösen Blut von etwa 0,2 bzw. 0,15l $O_2$/l Blut invasiv gefunden wurde und damit sich etwa $avDO_2$ = 0,05l $O_2$/l Blut im Normalfall ergibt. Demnach werden normalerweise nur ca. 25% der gesamten $O_2$-Bindungskapazität des Blutes bei der Passage durch die Gewebekapillaren ausgeschöpft:

$$SaO_{2\ arteriell} = 0,97 = \frac{20,4\ Vol.\text{-}\%}{21\ Vol.\text{-}\%}\ ,$$

(21 Vol.-% : $O_2$-Kapazität)

$$SaO_{2\ venös} = 0,73 = \frac{15,3\ Vol.\text{-}\%}{21\ Vol.\text{-}\%}\ ,$$

d.h.

$$avDO_2 = 20,4\ Vol.\text{-}\% - 15,3\ Vol.\text{-}\% \approx 5\ Vol.\text{-}\% \triangleq 0,05l\ O_2/l\ Blut$$

Als relative arteriovenöse $O_2$-Differenz ergibt sich demnach

$$SaO_{2\ arteriell} - SaO_{2\ venös} = 0{,}24$$

bzw.

$$avDO_{2\ rel} = \frac{5\ Vol.-\%}{21\ Vol.-\%} = 0{,}24.$$

Ausgehend vom Vorbekannten ist es daher Aufgabe der Erfindung, eine weiterentwickelte Vorrichtung und ein aussagefähigeres Arbeitsverfahren zum Betreiben der Vorrichtung zum Bestimmen und Auswerten von Funktionszustanden im Herz-Kreislauf-System anzugeben.

Die Lösung der Aufgabe erfolgt mit den Merkmalen der unabhängigen Patentansprüche, wobei die Unteransprüche mindestens zweckmäßige Ausgestaltungen und Weiterbildungen umfassen.

Die Erfindung geht von dem Grundgedanken aus, daß mit der in das Gewebe eingestrahlten Wellenlänge von 640nm die mit 840nm gemessene relative Blut- bzw. Erythrozytenmenge hinsichtlich ihres Oxygenierungsgrades erfaßt wird und sich somit für jede Herzaktion bzw. Atmungsperiode die erhaltenen Zeitfunktionen

$$x_1(t) = x_{NIR}(t),$$

$$x_2(t) = x_{ROT}(t)$$

ähnlich sind. Als entsprechendes Ähnlichkeitsmaß und damit als "Oxygenierungsgrad" (OX-Grad) bzw. Sättigungsgrad analog $SaO_2$ soll der Korrelationsfaktor $r(n) = OX(n)$, d.h. je Herzaktion bzw. Atmungsperiode n, definiert und abgeleitet werden. Es soll damit ebenso die arteriovenöse Differenz der $O_2$-Gehalte nichtinvasiv bestimmt werden. Es stellen dar:

$$x_1(t) = x_{NIR}(t) : \text{Bezugssignal, Sollwert (100\%ige Oxygenierung)}$$

$$x_2(t) = x_{ROT}(t) : \text{Istwert}$$

Zur Ermittlung der (linearen) Korrelation zwischen diesen Signalen wird die mittlere quadratische Abweichung $\overline{\varepsilon^2(t)}$ mit einem Kompensationsfaktor a wie folgt gebildet:

$$\overline{\varepsilon^2(t)} = \overline{[x_2(t) \cdot a - x_1(t)]^2} \rightarrow Min,$$

d.h. bei optimalem Kompensationsfaktor $a_{opt}$ muß "maximale Ähnlichkeit" erreicht sein, die mittlere quadratische Differenz wird dann minimal, so daß daraus der Korrelationsfaktor bestimmt werden kann. Man erhält

$$\overline{\varepsilon^2(t)} = a^2\ \overline{x_2^2(t)} + \overline{x_1^2(t)} - 2a\ \overline{x_1(t)x_2(t)}.$$

Durch Differentiation folgt

$$\frac{\delta\ \overline{\varepsilon^2(t)}}{\delta a} \quad bzw. \quad \frac{d\ \overline{\varepsilon^2(t)}}{da} = 0 = 2a_{opt} + \overline{x_2^2(t)} - 2\ \overline{x_1(t)x_2(t)},$$

bzw. erhält man für den optimalen Kompensationsfaktor

3

$$a_{opt} = \frac{\overline{x_1(t)\,x_2(t)}}{\overline{x_2^2(t)}} \quad .$$

Durch Einsetzen und Umformen folgt für die minimale mittlere quadratische Differenz

$$\overline{\epsilon^2(t)}_{min} = \overline{x_1^2(t)} \left[ 1 - \frac{\left(\overline{x_1(t)\cdot x_2(t)}\right)^2}{\overline{x_1^2(t)} \cdot \overline{x_2^2(t)}} \right]$$

bzw. in normierter Form

$$\frac{\overline{\epsilon^2(t)}_{min}}{\overline{x_1^2(t)}} = 1 - \frac{\overline{(x_1(t)\,x_2(t))}^2}{\overline{x_1^2(t)} \cdot \overline{x_2^2(t)}} \quad .$$

Übertragen auf die Verhältnisse bei der Sauerstoffsättigung im arteriellen und venösen Bereich sowie bei der nichtinvasiven Ermittlung der arteriovenösen Differenz der $O_2$-Gehalte wurde überraschend erkannt, daß: der Korrelationsfaktor

$$r = \frac{\overline{x_1(t)\cdot x_2(t)}}{\sqrt{\overline{x_1^2(t)} \cdot \overline{x_2^2(t)}}} = \frac{\overline{x_{NIR}(t)\cdot x_{ROT}(t)}}{\sqrt{\overline{x_{NIR}^2(t)} \cdot \overline{x_{ROT}^2(t)}}}$$

der arteriellen Sättigung $SaO_2$ entspricht. Dieser Wert wird nachfolgend "Oxygenierungsgrad" [OX-Grad] genannt. Er läßt sich bei Vorliegen entsprechender Volumenpulsationen je Herzaktion n bestimmen:

$$r(n) = OX(n) = \frac{\overline{x_{NIR}(n)\cdot x_{ROT}(n)}}{\sqrt{\overline{x_{NIR}^2(n)} \cdot \overline{x_{ROT}^2(n)}}} \quad .$$

Als mittlerer OX-Grad für eine bestimmte Meßdauer (Vorliegen von N Volumenpulsen bzw. N Herzaktionen) gilt:

$$\overline{r(n)} = \frac{1}{N} \sum_{n=1}^{N} r(n) = \overline{OX(n)} = \frac{1}{N} \sum_{n=1}^{N} OX(n)$$

$$\overset{\wedge}{=} \overline{SaO_2} \ .$$

Liegt eine extreme arterielle Durchblutungsstörung vor bzw. (allgemein ausgedrückt) kann nicht mehr von einer periodischen Volumenpulsation der Mikrozirkulation gesprochen werden, dann kann der Korrelationsfaktor als Mittelwert

$$r = \frac{\overline{x_{NIR}(t) \cdot x_{ROT}(t)}}{\sqrt{\overline{x^2_{NIR}(t)} \cdot \overline{x^2_{ROT}(t)}}} \ .$$

innerhalb der festgelegten Meßzeit (z.B. 60 s) bestimmt werden.

Wird dieses 2-Wellenlängen-Remissions-Verfahren auf die Verhältnisse bei der Lunge angewandt, indem der Sensor "lungennah" am Körper befestigt wird, so läßt sich je Atmungsperiode n* eine "arterielle Sättigung in der Lunge" (entspricht dem pulmonalen Venenblut) ermitteln

$$r(n*)_{Lunge} = \frac{\overline{x_{NIR}(n*) \cdot x_{ROT}(n*)}}{\sqrt{\overline{x^2_{NIR}(n*)} \cdot \overline{x^2_{ROT}(n*)}}} = OX(n*)_{Lunge}$$

bzw. als arithmetischer Mittelwert bei N* Atmungsperioden

$$\overline{r(n*)} \quad \frac{1}{N*} \sum_{n*=1}^{N*} r(n*) = \overline{OX(n*)} \quad .$$

Aus den ermittelten und dargestellten Verläufen r(n) [mit dem Mittelwert r(n)], r(n*) [mit dem Mittelwert r(n*)] sollen erfindungsgemäß die absoluten und relativen Standardabweichungen sowie die in den Verläufen auftretenden mittleren Perioden in den entsprechenden Autokorrelationsfunktionen als quasi Gütemaße im Herz-Kreislauf-System bestimmt werden.

Bezieht man die Standardabweichung (Streuung) $S_{\Delta OX}$

$$S_{\Delta OX} = \sqrt{\frac{1}{N-1} \sum_{n=1}^{N} [OX(n) - \overline{OX(n)}]^2}$$

auf den Mittelwert $\overline{OX(n)}$, so soll eine "Oxygenierungsvariabilität" OXV eingeführt werden:

$$OXV = \frac{S_{\Delta OX}}{\overline{OX(n)}} \; .$$

Eine solche Kenngröße wird auch für die Lunge ermittelt.
Die minimale mittlere quadratische Differenz $\overline{\epsilon^2(t)}_{min}$ bzw. in normierter Form

$$\frac{\overline{\epsilon^2(t)}_{min}}{\overline{x^2}_{NIR}(t)}$$

läßt sich je Herzaktion n ermitteln und entspricht dem Quadrat
der absoluten und relativen arteriovenösen Differenz der $O_2$-Gehalte:

$$\overline{avDO_2} \; \hat{=} \; \overline{D(n)}$$

bzw.

$$D(n) = \sqrt{\overline{\epsilon^2(t)}(n)} = \sqrt{\overline{x^2}_{NIR}(t)(n)} \; \sqrt{1-OX^2(n)} \; .$$

Als relative arteriovenöse Different folgt

$$d(n) = \frac{D(n)}{\overline{x^2_{NIR}}(n)} = \sqrt{1 - OX^2(n)} \; .$$

Als Näherung wird daraus definiert

$$\overline{avDO_2} \; \hat{=} \; \overline{D(n)}$$

$$\hat{=} \; O_2\text{-Kapazität} \cdot \sqrt{1 - (SaO_2)^2}$$

$$\frac{\overline{avDO_2}}{O_2\text{-Kapazität}} = \sqrt{1 - (SaO_2)^2} \; .$$

Die Erfindung soll anhand von Ausführungsbeispielen und von Figuren näher erläutert werden. Hierbei zeigen:

Fig. 1    ein aus einem Modell abgeleiteter Zusammenhang zwischen der absoluten und relativen arteriovenösen

$O_2$-Differenz sowie des absoluten und relativen arteriellen $O_2$-Gehaltes,

Fig. 2 den Oxygenierungsgrad vor [a)] und nach einer Infusion von Prostavasin bei einem liegenden Patienten mit pAVK (Meßort Großzehe) und Ruheatmung,

Fig. 3 den Oxygenierungsgrad bei einem 79jährigen liegenden Patienten mit Linksherzinsuffizienz und bei Ruheatmung (Meßort 2. Finger re.),

Fig. 4 die integrale Mikrozirkulation, gemessen mit NIR-ROT-Remissions-Photoplethysmographie, am Meßort Großzehe re. bei einem 85jährigen Patienten mit pAVK,

Fig. 5 Remissions-Photoplethysmographie-Signale bei Lungenatmung mit überlagerten Volumenpulsationen am Meßort Oberkörper/Lungennähe,

Fig. 6a den Zeitausschnitt einer an einer Fingerbeere gemessenen NIR-Blutvolumenpulsation einschließlich markierte Herzperiodendauern [$T_H$(n) zum n-ten Volumenpuls gehörend usw.],

Fig. 6b ein Tachogramm der Herzperiodendauer $T_H$ in Abhängigkeit des entsprechenden Volumenpulses n als Beispiel,

Fig. 7 ein Tachogramm $T_H$=f(n) für einen 40jährigen Normalprobanden,

Fig. 8 die zu Fig. 7 gehörende Autokorrelationsfunktion $\Phi_{\Delta TH}$(m) einschließlich Kenn- und Normalwerte,

Fig. 9 ein aus Fig. 7 abgeleitetes Histogramm der Herzperiodendauern einschließlich definierte Grenzen sowie prozentuale Angaben des Auftretens der Perioden in den entsprechenden Bereichen,

Fig.10 ein Tachogramm $T_H$=f(n) bei einer 35jährigen Diabetikerin (Typ I WHO),

Fig.11 die zu Fig. 10 gehörende AKF $\Phi_{\Delta TH}$(m),

Fig.12 ein Tachogramm $T_H$=f(n) bei einer 72jährigen liegenden Patientin bei Ruheatmung mit koronarer 3-Gefäßerkrankung, arterieller Hypertonie und Hinterwandinfarkt,

Fig.13 die zu Fig. 12 gehörende AKF $\Phi_{\Delta TH}$(m),

Fig.14 Die Blutvolumenpulsation des n-ten sowie (n+1)-ten Pulses mit den zugehörigen Flächen $F_n$ und $F_{n+1}$ als Maß für die mikrovaskuläre Perfusion an der Meßstelle, einem Mikrogefäßbereich,

Fig.15 den Verlauf des relativen Füllungsvolumens (Meßort 2. Fingerbeere re.) bei dem Normalprobanden nach Fig. 9,

Fig.16 den Verlauf des relativen Füllungsvolumens (Meßort 2. Fingerbeere re.) bei dem Diabetiker nach Fig. 10, und

Fig.17 den Verlauf des Oxygenierungsgrades OX=f(n) bei dem Normalprobanden nach Fig. 15.

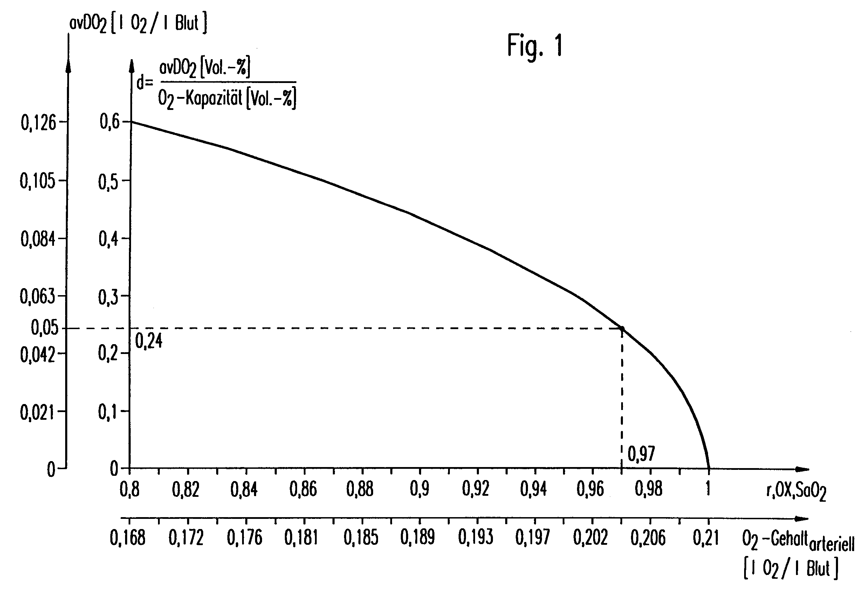

Fig. 1 zeigt die graphische Darstellung der erwähnten Abhängigkeiten. Es ist ersichtlich, daß bei Oxygenierungsgraden (Sättigungsgraden) von etwa Eins die arteriovenöse Differenz nach Null verläuft, d.h. das arterielle Blut dann nicht entsättigt wird. Die invasiv ermittelten Normalwerte

$$SaO_{2arteriell} = 0{,}97{,}$$

$$SaO_{2arteriell} - SaO_{2venös} = 0{,}24$$

liegen direkt auf dem theoretisch ermittelten Kurvenverlauf.

Die in Fig. 1 angegebenen Maßstäbe für die Absolutwerte bei $O_2$-Gehalt$_{arteriell}$ sowie av$DO_2$ in [l $O_2$/l Blut] beruhen auf einer $O_2$-Kapazität von 21 Vol.-% [nach Rein, H.; Schneider, M.: Physiologie des Menschen, 11. Aufl., Springer Berlin/Göttingen/Heidelberg 1955, S. 39].

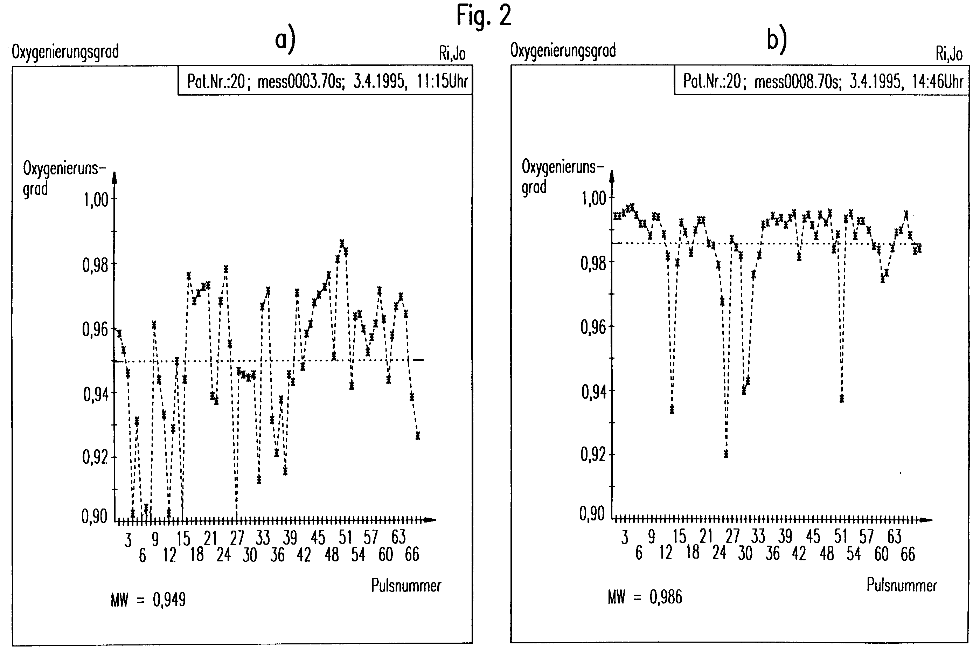

Fig. 2 zeigt die Verläufe der Oxygenierungsgrade am Meßort Großzehe vor [a)] und nach [b)] Infusionen von Prostavasin bei einem 72jährigen Patienten mit peripherer arterieller Verschlußkrankheit (pAVK). Neben einer Erhöhung des mittleren Oxygenierungsrades durch diese Infusion wird die Verringerung der Oxygenierungsvariabilität deutlich, z.T. treten atmungsdominante Perioden von 4 bis 5 Herzschlägen auf.

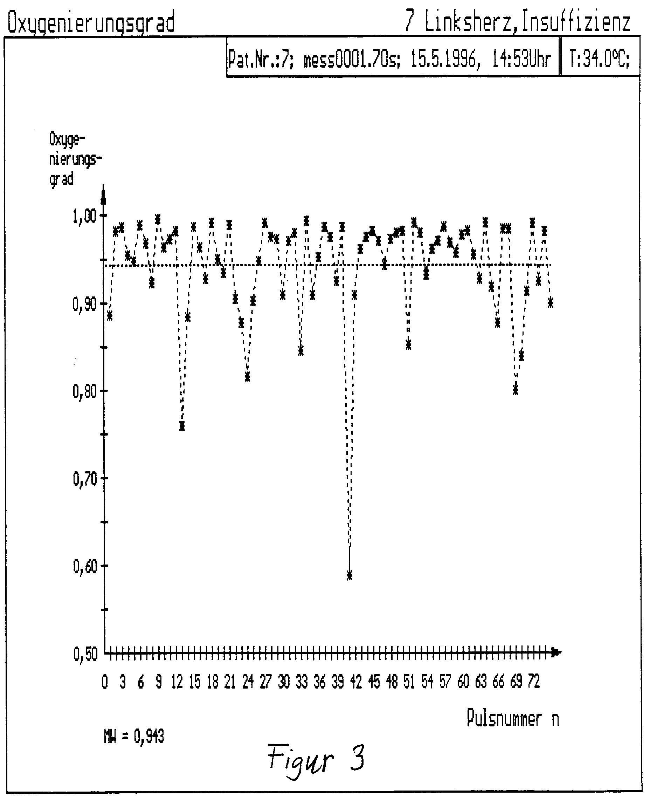

Aus Fig. 3 gehen die Oxygenierungsverhältnisse mit z.T. auftretenden atmungsdominanten Perioden bei einem 79jährigen liegenden Patienten mit Linksherzinsuffizienz und Ruheatmung hervor (Meßort 2. Fi. li).

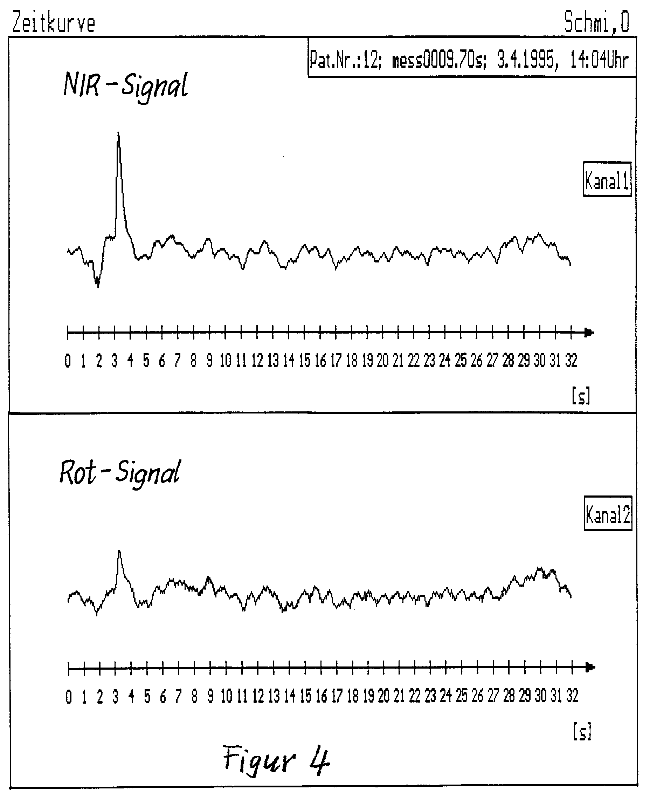

Fig. 4 zeigt die Verläufe der mit NIR-ROT-Remissions-Photoplethysmographie gemessenen integralen Mikrozirkulation am Meßort Großzehe bei einem 85jährigen Patienten mit pAVK. Da kaum von periodischen Volumenpulsationen gesprochen werden kann, wird bei solchen Verläufen der Zeitfunktionen nur der Mittelwert des Oxygenierungsgrades (Korrelationsfaktors)

$$\overline{OX} = \frac{\overline{x_{NIR}(t) \cdot x_{ROT}(t)}}{\sqrt{\overline{x^2_{NIR}(t)} \cdot \overline{x^2_{ROT}(t)}}}$$

bestimmt.

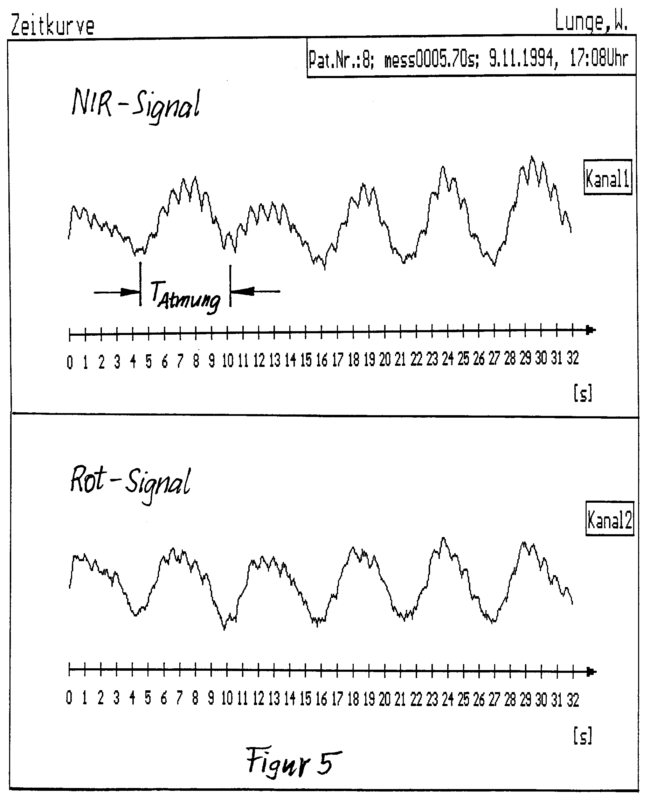

In Fig. 5 sind die bei einem Normalprobanden ermittelten Remissions-Photoplethysmographie-Signale bei Lungenatmung mit überlagerten Volumenpulsationen am Meßort (Lungennähe) dargestellt. Der während einer Atmungsperiode $T_{Atmung}$ sich einstellende Oxygenierungsgrad $OX(n^*)_{Lunge}$ wird analog zu $OX(n)$ bestimmt. Zur Unterdrückung der höherfrequenten Volumenpulsationen im NIR- und ROT-Signal wird jeweils der gleiche Tiefpaß mit einer Grenzfrequenz von ca. 0,6 Hz diesen Signalen nachgeschaltet, um nachfolgend den Oxygenierungsgrad $OX(n^*)_{Lunge}$ zu ermitteln.

Mit der Nahen-Infrarot-Remissions-Photoplethysmographie läßt sich, wie aus DE 42 38 641 bekannt, nichtinvasiv an einem entsprechenden peripheren Ort (z.B. Fingerbeere) die relative Menge des Blutes je Herzschlag bei einer ausgewählten Meßzeit erfassen, die das Mikrogefäßsystem in Form von Volumenpulswellen passiert.

Da die Volumenpulsation an einer "Endstelle" des arteriellen Gefäßsystems gemessen wird, enthält sie bekanntlich alle wesentlichen Parameter, die das Zusammenwirken der "Zentrale", dem Herzen, mit dem dazwischen liegenden Gefäßsystem charakterisieren:

- Herzperiodendauer $T_H$ und deren Veränderung je Herzpulsation während der Meßzeit (z.B. 60 s). In "Perfusion" 7/96, 9. Jahrg., Seiten 268 bis 279, wird dargelegt, daß im Normalfall (keine pAVK auf der "Übertragungsstrecke", keine Stenosen) die dort gemessene Pulsperiodendauer mit der Herzperiodendauer $T_H$ mit ausreichender Näherung gleichgesetzt werden kann. Als Meßort ist deshalb im allgemeinen die Fingerbeere zu wählen, falls dort keine Durchblutungsstörungen auftreten (Cave: niedrigerer Blutdruck auf der Seite der Messung).
- Parameter des Mikro- und arteriellen Makrogefäßbereiches, wie u.a. das relative Füllungsvolumen FV oder der Oxygenierungsgrad bzw. die arteriovenöse Differenz der $O_2$-Gehalte.

Hierzu sei auf die Veröffentlichungen "Perfusion" 7/96, 9. Jahrgang, Seite 268 bis 279, "Perfusion" 8/96, 9. Jahrg., Seiten 300 bis 310, sowie "European Journal of Medical Research", Vol. 1, 22. Februar 1996, Seiten 237 bis 244 verwiesen, die zur Erläuterung des hier vorgestellten Gegenstandes als zugehörig betrachtet werden.

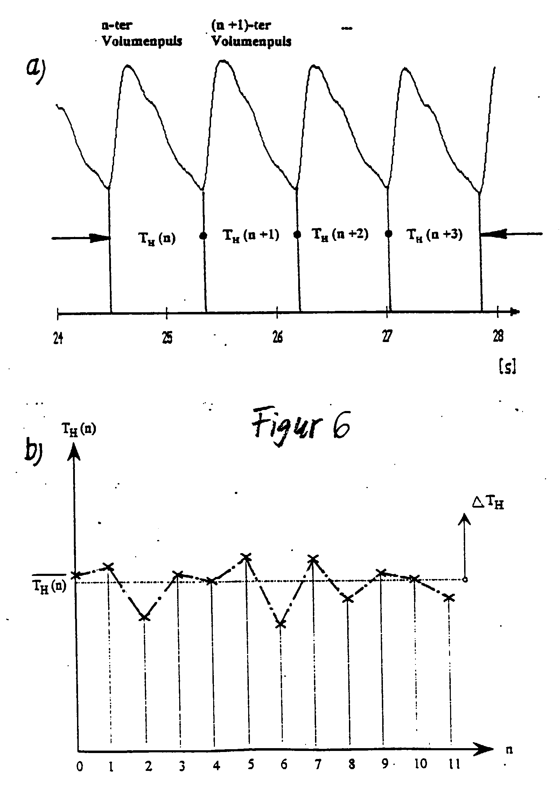

Fig. 6a zeigt den Zeitausschnitt einer an einer Fingerbeere gemessenen NIR-Blutvolumenpulsation einschließlich markierte Herzperiodendauern [$T_H(n)$ zum n-ten Volumenpuls gehörend usw.] sowie Fig. 6b das daraus abgeleitete Tachogramm $T_H(n)$ als Beispiel. Ersichtlich ist das Auftreten von Herzperiodendauerabweichungen $\Delta T_H(n)$ vom Mittelwert $\overline{T_H(n)}$.

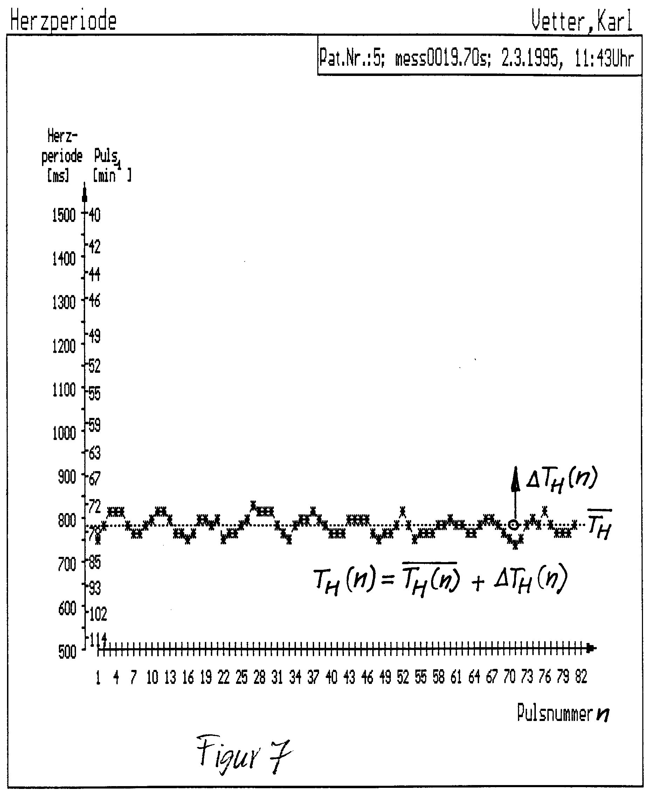

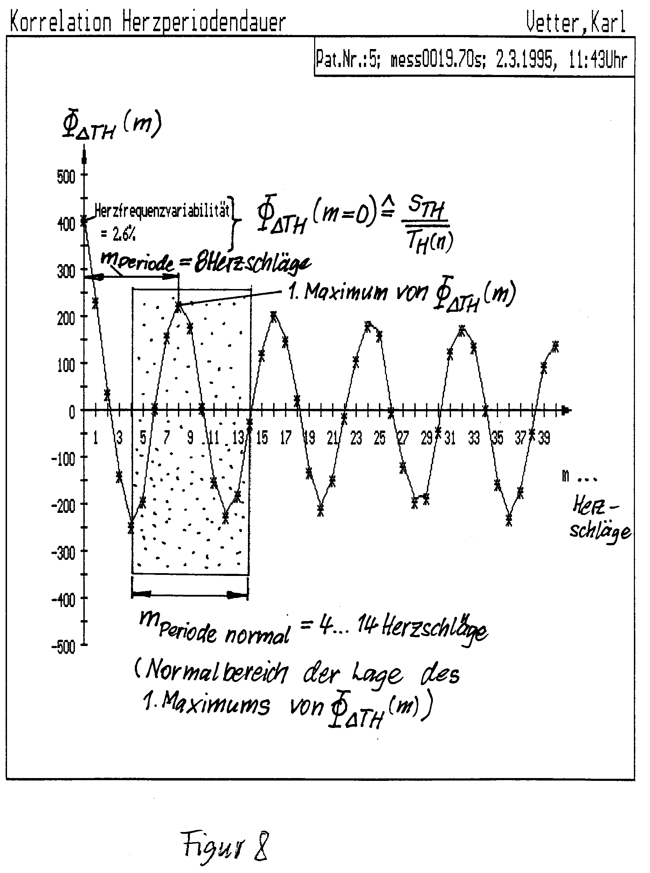

In Fig. 7 ist das Tachogramm $T_H = f(n)$ bei einem 40jährigen Normalprobanden dargestellt, wo die respiratorische Sinusarrhythmie deutlich wird, während Fig. 8 die zur Herzperiodendauerabweichung $\Delta T_H(n)$ zugehörige Autokorrelationsfunktion $\Phi_{\Delta TH}(m)$ als Mittelwertfunktion einschließlich Normalbereich sowie in $\Phi_{\Delta TH}(m)$ auftretende Kennwerte (u.a. respiratorische Sinusarrhythmie mit 8 Herzschlägen im Mittel) wiedergibt.

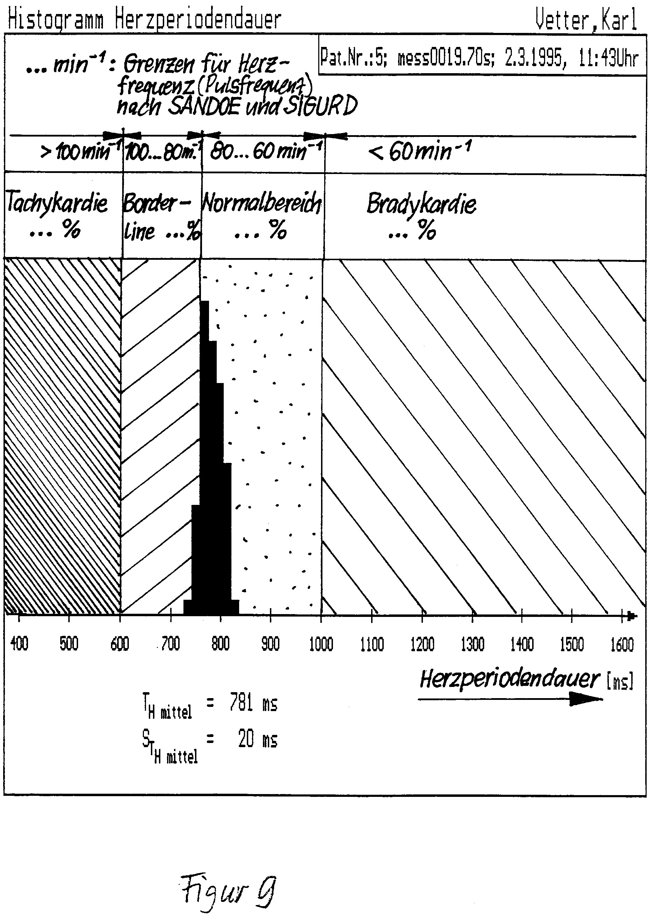

Fig. 9 zeigt das für den Normalprobanden nach Fig. 7 sich ergebende Histogramm der Herzperiodendauer einschließlich definierte Grenzen mit prozentualen Angaben des Auftretens der Perioden in den entsprechenden Bereichen.

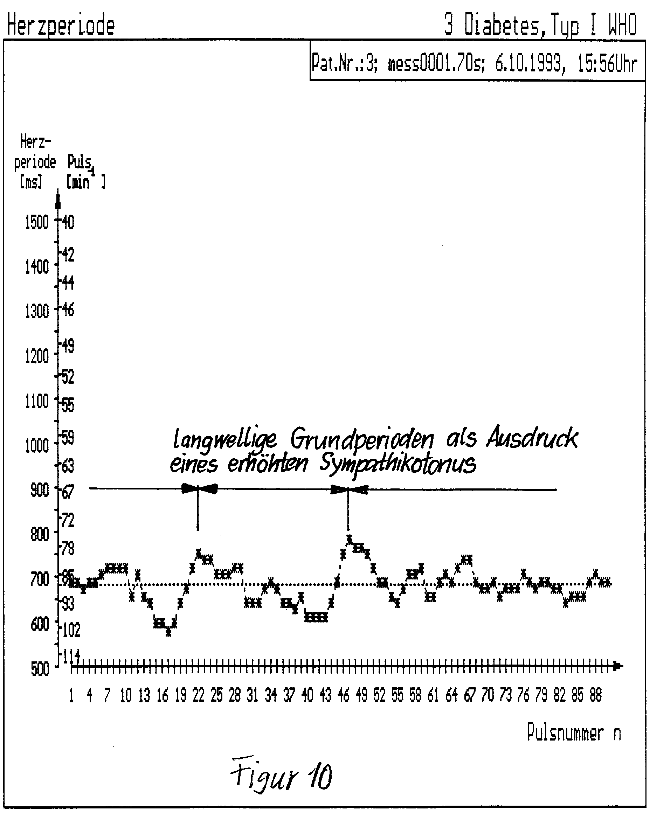

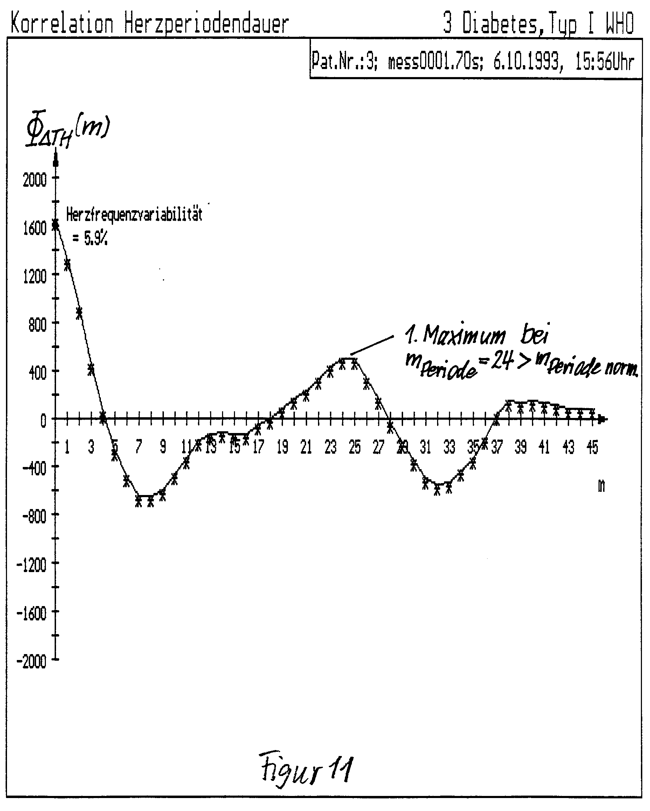

Die Fig. 10 und 11 geben die kardiovaskulären Kennfunktionen bei einer 35jährigen Diabetikerin (Typ I WHO) wieder. Das Auftreten von anormalen langen Grundperioden als Ausdruck eines erhöhten Sympathikotonus bzw. einer veränderten Herzmikrozirkulation ist deutlich.

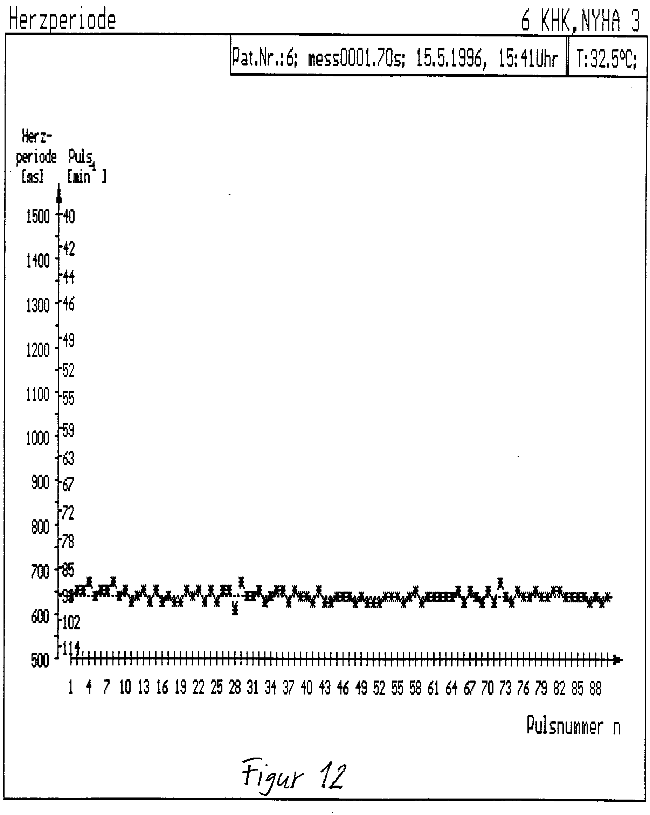

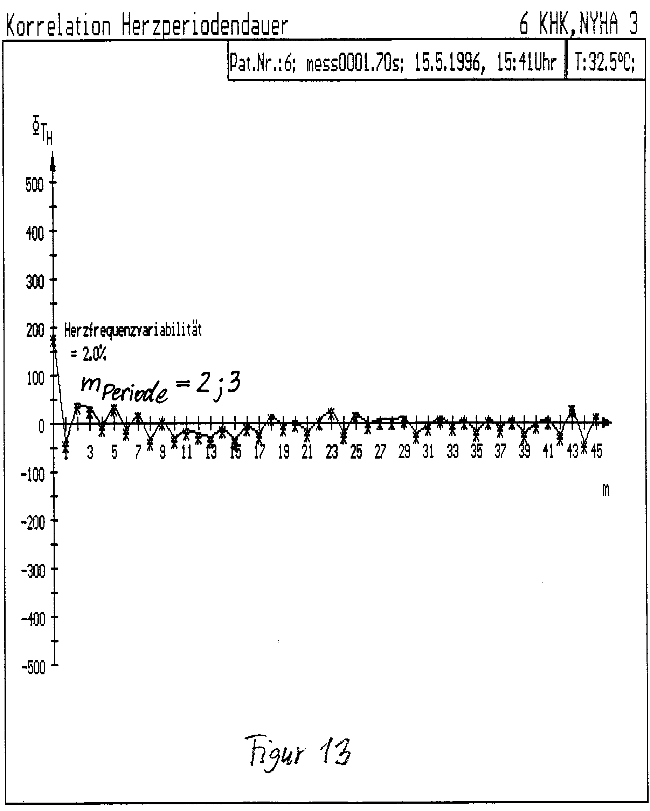

Die Fig. 12 und 13 zeigen die Verläufe bei einer 72jährigen liegenden Patientin bei Ruheatmung mit koronarer 3-Gefäßerkrankung, arterieller Hypertonie und Hinterwandinfarkt. Die Unterschiede zum Normalprobanden nach den Fig. 7, 8 werden ebenso deutlich wie zur Typ I-Diabetikerin nach den Fig. 10, 11. Charakteristisch für den Verlauf nach Fig. 13 ist die geringe Herzfrequenzvariabilität, insbesondere das Auftreten von Perioden von 2 bzw. 3 Herzschlägen.

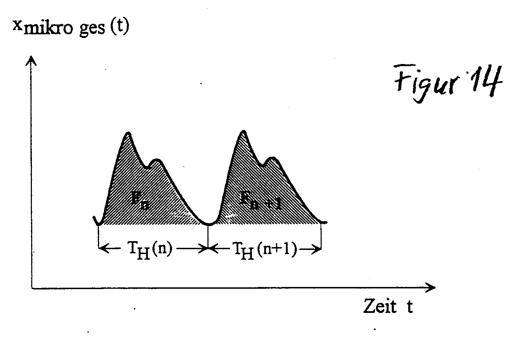

Als "relatives (mikrovaskuläres) Füllungsvolumen" an einer entsprechenden Meßstelle (einem Mikrogefäßbereich) wird die Fläche eines jeden Volumenpulses, bezogen auf den mittleren Flächeninhalt aller Pulsationen in einem Meßfile (z.B. 80 Volumenpulse) ermittelt, wie Fig. 14 zeigt.

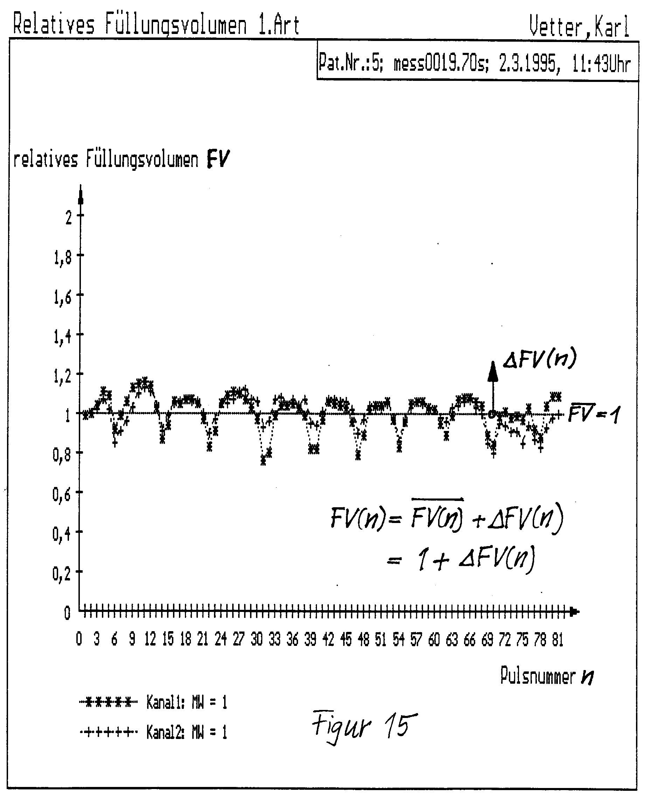

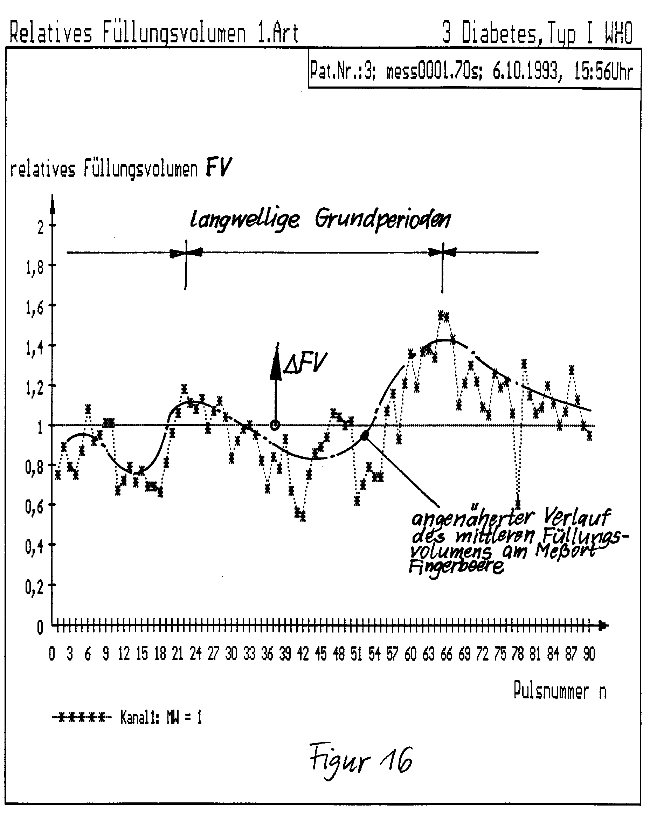

Fig. 15 zeigt den Verlauf eines solchen relativen Füllungsvolumens (Meßstelle 2. Fingerbeere re.) bei dem Normalprobanden nach Fig. 7, Fig. 16 den für den Diabetiker nach Fig. 10 (Meßstelle 2. Fingerbeere re.).

Erfindungsgemäß wurde erkannt, daß analog zum Tachogramm $T_H = f(n)$ ein Tachogramm des relativen Füllungsvolumens FV(n) bestimmt werden kann und daraus eine Autokorrelationsfunktion der Abweichung des relativen Füllungsvolumens vom Mittelwert FV1=1 analog zu $\Phi_{\Delta TH}(m)$ ermittelt werden kann:

$$\Delta FV(n) = FV(n) - \overline{FV(n)}$$
$$= FV(n) - 1$$

Damit folgt analog $\Phi_{\Delta TH}(m)$:

$$\Phi_{\Delta FV}(m) = \overline{FV(n) \cdot FV(n-m)} - 1 \; .$$

Bei m=0 erhält man die "Füllungsvolumenvariabilität" FVV

$$FVV = \sqrt{\Phi_{\Delta FV}(0)},$$

während aus $\Phi_{\Delta FV}(m)$ die mittleren Füllungsperioden (z.B. Atmungsdominanz im Normalfall) ersichtlich sind.

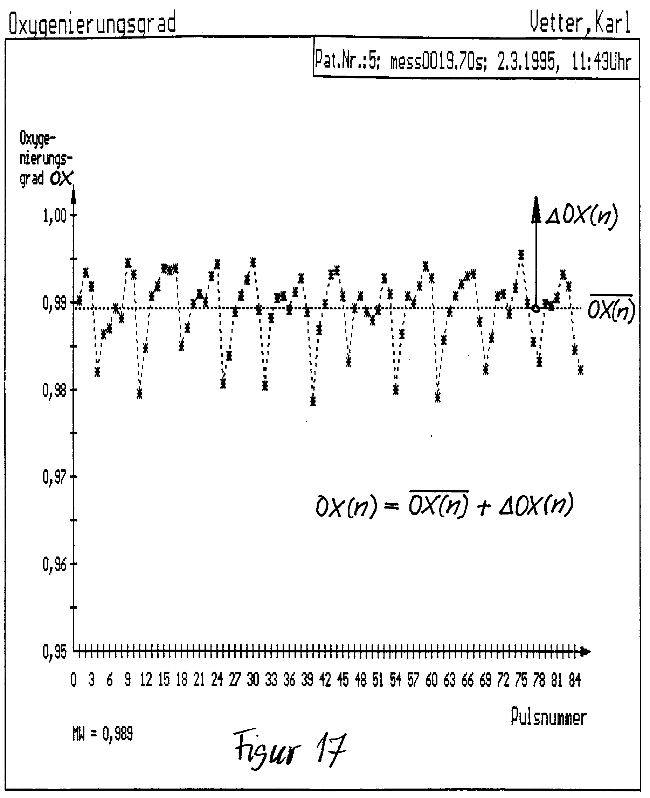

Erfindungsgemäß wurde des weiteren erkannt, daß ebenso aus dem Tachogramm des Oxygenierungsgrades OX=f(n) eine Autokorrelationsfunktion der Abweichung des Oxygenierungsgrades vom Mittelwert $\overline{OX(n)}$ abgeleitet werden kann, wie in Fig. 17 für den Normalprobanden nach Fig. 15 gezeigt wird.

Es ergibt sich mit

$$\Delta OX(n) = OX(n) - \overline{OX(n)}$$

für die Autokorrelationsfunktion analog $\Phi_{\Delta TH}(m)$:

$$\Phi_{\Delta OX}(m) = \overline{OX(n) \cdot OX(n-m)} - \overline{[OX(m)]}^2 \; .$$

Ebenfalls folgt aus $\Phi_{\Delta OX}(m=0)$ die "Oxygenierungsgradvariabilität" OXV

$$OXV \;\; = \;\; \frac{\sqrt{\Phi_{\Delta OX}(m=0)}}{\overline{OX(n)}} \;\; ,$$

aus dem Verlauf $\Phi_{\Delta OX}(m)$ die auftretenden mittleren Perioden (z.B. Atmungsdominanz im Normalfall sowie bei Vorliegen von Perfusionsgleichgewicht, siehe Fig. 17).

In einer weiteren Ausführungsform der Erfindung werden die eingeführten Zustandsfunktionen $T_H$=f(n) , FV= f(n) , OX=f(n) sowie

$$\frac{T_H}{FV} = f(n)$$

untereinander kreuzkorreliert, d.h. Korrelationsfaktoren sowie Perioden $m_{Periode}$ nach dem Bildungsalgorithmus

$$\Phi xy(m) = \overline{x(n) \cdot y(n-m)}$$

bestimmt:

$\Phi_{TH,FV}(m),$
$\Phi_{FV,OX}(m),$

$$\Phi_{\frac{FV}{TH},OX}(m)$$

$\Phi_{TH,OX}$ (m).

Ebenfalls erfolgt ein Vergleich mit Normalverläufen und -kennwerten.

**Patentansprüche**

1. Vorrichtung zum Bestimmen und Auswerten von Funktionszuständen im Herz-Kreislauf-System, insbesondere der Blutgefäße einschließlich des Herzens im Zusammenwirken mit dem autonomen Nervensystem, gekennzeichnet durch

   - Mittel zum Erfassen der Zustandsfunktionen durch nichtinvasive optische und/oder elektrische Messung,
   - Zustandsbeschreibung je Herzaktion und daraus abgeleiteter Auto- und/oder Kreuzkorrelationsfunktionen als verallgemeinerte Mittelwertfunktionen bzw. zugehöriger Amplitudenspektren als Zustandsfunktionen,
   - Mittel zum Speichern und Darstellen der Zustandsfunktionen und
   - Mittel zum Vergleichen der erhaltenen Zustandsfunktionen mit Normalwerten und -verläufen.

2. Vorrichtung nach Anspruch 1,
   dadurch gekennzeichnet,
   daß Mittel zur Bestimmung des Oxygenierungsgrades des Blutes auf der Basis einer Zwei-Wellenlängen-NIR-ROT-Remissions-Photoplethysmographiemessung vorgesehen sind, wobei die Zeitfunktionen

   $$x_1(t) = x_{NIR}(t) \text{ und}$$

   $$x_2(t) = x_{ROT}(t) \text{ je Herzaktion bestimmt werden}$$

   und das Ähnlichkeitsmaß der Zeitfunktionen sowie der Korrelationsfaktor ermittelt wird, wobei der Korrelationsfaktor sich aus

   $$r = \frac{\overline{x_1(t)\ x_2(t)}}{\sqrt{\overline{x_1^2(t)} \cdot \overline{x_2^2(t)}}} = \frac{\overline{x_{NIR}(t) \cdot x_{ROT}(t)}}{\sqrt{\overline{x_{NIR}^2(t)} \cdot \overline{x_{ROT}^2(t)}}}$$

   ergibt und als Oxygenierungsgrad festgelegt ist.

3. Arbeitsverfahren zum Betreiben der Vorrichtung nach Anspruch 1 oder 2,
   dadurch gekennzeichnet,
   daß aus den ungestörten Volumenpulsationen des NIR-Signals bei Transmissions- oder Remissionsphotoplethysmographie sowie bei der elektronischen Oszillographie bei einer vorgegebenen Meßzeit das Herzperiodendauer-Tachogramm $T_H = f(n)$, d.h. je Herzaktion n, abgeleitet und dargestellt wird, um Perioden und Abweichungen vom Mittelwert $\overline{T_H(n)}$ zu bestimmen.

4. Arbeitsverfahren nach Anspruch 2,
   dadurch gekennzeichnet,
   daß aus der Abweichung der Herzperiodendauer vom linearen Mittelwert je Herzaktion n

   $$\Delta T_H(n) = T_H(n) - \overline{T_H(n)}$$

   deren Autokorrelationsfunktion

   $$\Phi_{\Delta TH}(m) = \overline{\Delta T_H(n) \cdot \Delta T_H(n-m)}$$

   gebildet wird, um daraus die in der Zeitreihe $T_H = f(n)$ auftretenden mittleren Perioden m als Vielfaches der Herzfrequenz darzustellen, und die bei m=0 auftretende Standardabweichung $S_{TH}$ als mittlere quadratische Abweichung der Herzperiodendauer bzw. durch Bezug auf den linearen Mittelwert $\overline{T_H(n)}$ die Herzfrequenzvariabilität

VHF

$$VHF = \frac{S_{TH}}{\overline{T_H(n)}} \cdot 100 \ [\%] \quad \text{zu ermitteln.}$$

5. Arbeitsverfahren nach Anspruch 4,
dadurch gekennzeichnet,
daß im Bildungsalgorithmus von $\Phi_{\Delta TH}(m)$ im Tachogramm $T_H = f(n)$ auftretende Extrasystolen durch den Mittelwert $\overline{T_H(n)}$ ersetzt werden.

6. Arbeitsverfahren nach Anspruch 4,
dadurch gekennzeichnet,
daß bei Vorliegen von Perfusionsgleichgewicht der erhaltene Verlauf $\Phi_{\Delta TH}(m)$ mit Normalverläufen ($m_{Periode \ normal}$ = 4...14 Herzschläge) verglichen wird.

7. Arbeitsverfahren nach den Ansprüchen 4 oder 6,
dadurch gekennzeichnet,
daß in $\Phi_{\Delta TH}(m)$ auftretende Perioden ($m_{Periode}$ = 2;3; >14 Herzschläge) Nichtnormalverläufe darstellen.

8. Arbeitsverfahren nach einem der Ansprüche 4, 6 oder 7,
dadurch gekennzeichnet,
daß zur Charakterisierung des kardiovaskulären Zustandes neben der Zustandsfunktion $\Phi_{\Delta TH}(m)$ die weiteren Kennwerte bzw. -funktionen systolischer und diastolischer Blutdruck, Oxygenierungsgrad einschließlich dessen Autokorrelationsfunktion $\Phi_{\Delta OX}(m)$ und/oder relatives Füllungsvolumen FV einschließlich dessen Autokorrelationsfunktion $\Phi_{\Delta FV}(m)$ Verwendung finden.

9. Arbeitsverfahren nach den Ansprüchen 1, 3 oder 4,
dadurch gekennzeichnet,
daß als bezogene Funktionstests des kardiovaskulären Systems die tiefe Atmung zur Erhöhung der Förderleistung des Herzens sowie die reflektorische Vasokonstriktion durch Sympathikusreizung benutzt werden und sich dadurch im Normalfall Veränderungen der Kennfunktionen $T_H = f(n)$, $\Phi_{\Delta TH}(m)$ ergeben, die zur Bewertung heranziehbar sind.

10. Arbeitsverfahren nach Anspruch 4,
dadurch gekennzeichnet,
daß die Autokorrelationsfunktion $\Phi_{\Delta TH}(m)$ aus dem RR-Abstand im EKG gebildet wird.

11. Arbeitsverfahren nach den Ansprüchen 3 oder 4,
dadurch gekennzeichnet,
daß durch Fouriertransformation von $\Phi_{\Delta TH}(m)$ das gleichwertige Leistungsspektrum der Herzfrequenzvariabilität gebildet und als Vergleichswert zur Auswertung herangezogen wird.

12. Arbeitsverfahren nach einem der vorangegangenen Ansprüche,
dadurch gekennzeichnet,
daß aus dem Tachogramm des relativen Füllungsvolumens FV(n) die Autokorrelationsfunktion als Zustandsfunktion

$$\Phi_{\Delta FV}(m) = \overline{FV(n) \cdot FV(n-m)} - 1$$

ermittelt wird, woraus bei m=0 die "Füllungsvolumenvariabilität" FVV sowie aus $\Phi_{\Delta FV}(m)$ die auftretenden mittleren Perioden ableitbar sind.

13. Arbeitsverfahren nach Anspruch 12
dadurch gekennzeichnet,

daß bei Vorliegen von Perfusionsgleichgewicht der erhaltene Verlauf $\Phi_{\Delta FV}(m)$ mit Normalverläufen verglichen und zur Auswertung herangezogen wird.

14. Arbeitsverfahren nach Anspruch 12,
dadurch gekennzeichnet,
daß die bei tiefer Atmung sich ergebende erhöhte Förderleistung des Herzens Veränderungen von FV(n) sowie von $\Phi_{\Delta FV}(m)$ hervorruft, die quantitativ mit den Kennwerten von $\Phi_{\Delta FV}(m)$ ermittelt und zur Auswertung herangezogen werden.

15. Arbeitsverfahren nach Anspruch 1, 2, 4,
dadurch gekennzeichnet,
daß aus dem Tachogramm des Oxygenierungsgrades OX(n) die Autokorrelationsfunktion als Zustandsfunktion

$$\Phi_{\Delta OX}(m) = \overline{OX(n) \cdot OX(n\text{-}m)} - \overline{[OX(n)]}^{\,2}$$

ermittelt wird, woraus bei m=0 eine Oxygenierungsgradvariabilität OXV sowie aus $\Phi_{\Delta OX}(m)$ die auftretenden mittleren Perioden abgeleitet und zur Auswertung herangezogen werden.

16. Arbeitsverfahren nach Anspruch 15,
dadurch gekennzeichnet,
daß bei Vorliegen des Perfusionsgleichgewichts der erhaltene Verlauf $\Phi_{\Delta OX}(m)$ mit Normalverläufen verglichen und zur Auswertung herangezogen wird.

17. Arbeitsverfahren nach Anspruch 1,
dadurch gekennzeichnet,
daß aus dem Tachogramm $\frac{TH}{FV}$ (n) die Autokorrelationsfunktion

$$\Phi_{\Delta \frac{TH}{FV}}(m)$$

als Zustandsfunktion der Peripherie des Herz-Kreislaufsystems ermittelt wird nach:

$$\Phi_{\Delta \frac{TH}{FV}}(m) = \overline{\Delta \frac{TH}{FV}(n) \cdot \Delta \frac{TH}{FV}(n\text{-}m)},$$

woraus bei m=0 eine Herz-Peripherie-Variabilität sowie aus

$$\Phi_{\Delta \frac{FV}{TH}}(m)$$

die auftretenden mittleren Perioden bestimmt und zur Auswertung mit Normalverläufen verglichen werden.

18. Verfahren zum Feststellen der Auswirkungen von Pharmaka oder Drogen sowie zum Ableiten von Größen zur Patientenüberwachung unter Nutzung der Zustands- und Auswertungsparameter erhalten nach einem oder mehreren der Ansprüche 1 bis 17.

Fig. 1

EP 0 849 686 A2

# Fig. 2

**a)**

Oxygenierungsgrad                                        Ri,Jo

Pat.Nr.:20; mess0003.70s; 3.4.1995, 11:15Uhr

Oxygenierungs-grad

1,00

0,98

0,96

0,94

0,92

0,90

3   9   15   21   27   33   39   45   51   57   63
  6   12   18   24   30   36   42   48   54   60   66

Pulsnummer

MW = 0,949

**b)**

Oxygenierungsgrad                                        Ri,Jo

Pat.Nr.:20; mess0008.70s; 3.4.1995, 14:46Uhr

Oxygenierungs-grad

1,00

0,98

0,96

0,94

0,92

0,90

3   9   15   21   27   33   39   45   51   57   63
  6   12   18   24   30   36   42   48   54   60   66

Pulsnummer

MW = 0,986

EP 0 849 686 A2

Oxygenierungsgrad                          7 Linksherz,Insuffizienz

Figur 3

Zeitkurve                                              Schmi, O

Pat.Nr.:12; mess0009.70s; 3.4.1995, 14:04Uhr

NIR - Signal

Kanal1

0 1 2 3 4 5 6 7 8 9 10 11 12 13 14 15 16 17 18 19 20 21 22 23 24 25 26 27 28 29 30 31 32

[s]

Rot - Signal

Kanal2

0 1 2 3 4 5 6 7 8 9 10 11 12 13 14 15 16 17 18 19 20 21 22 23 24 25 26 27 28 29 30 31 32

[s]

Figur 4

Zeitkurve                                                    Lunge, W.

Pat.Nr.:8; mess0005.70s; 9.11.1994, 17:08Uhr

NIR-Signal

Kanal1

$T_{Atmung}$

0 1 2 3 4 5 6 7 8 9 10 11 12 13 14 15 16 17 18 19 20 21 22 23 24 25 26 27 28 29 30 31 32

[s]

Rot-Signal

Kanal2

0 1 2 3 4 5 6 7 8 9 10 11 12 13 14 15 16 17 18 19 20 21 22 23 24 25 26 27 28 29 30 31 32

[s]

Figur 5

Figur 6

Figur 7

Figur 8

Histogramm Herzperiodendauer                    Vetter, Karl

... min⁻¹ : *Grenzen für Herz-*
*frequenz (Pulsfrequenz)*
*nach SANDOE und SIGURD*

Pat.Nr.:5; mess0019.70s; 2.3.1995, 11:43Uhr

> 100 min⁻¹ | 100...80 m⁻¹ | 80...60 min⁻¹ | < 60 min⁻¹

| Tachykardie ... % | Border- Line ...% | Normalbereich ... % | Bradykardie ... % |

400   500   600   700   800   900   1000   1100   1200   1300   1400   1500   1600

*Herzperiodendauer* [ms]

$T_{H\,mittel} = 781\ ms$

$S_{T_{H\,mittel}} = 20\ ms$

Figur 9

21

Figur 10

Korrelation Herzperiodendauer  3 Diabetes, Typ I WHO

Pat.Nr.:3; mess0001.70s; 6.10.1993, 15:56Uhr

$\bar{\Phi}_{\Delta TH}(m)$

Herzfrequenzvariabilität = 5.9%

1. Maximum bei $m_{Periode} = 24 > m_{Periode\ norm.}$

$m$

Figur 11

Figur 12

Korrelation Herzperiodendauer                                      6 KHK,NYHA 3

Pat.Nr.:6; mess0001.70s; 15.5.1996, 15:41Uhr   T:32.5°C;

Herzfrequenzvariabilität = 2.0%

$m_{Periode} = 2;3$

Figur 13

Figur 14

Relatives Füllungsvolumen 1.Art          Vetter,Karl

Pat.Nr.:5; mess0019.70s; 2.3.1995, 11:43Uhr

relatives Füllungsvolumen **FV**

$$FV(n) = \overline{FV(n)} + \Delta FV(n)$$
$$= 1 + \Delta FV(n)$$

Pulsnummer **n**

-*****- Kanal1: MW = 1

-+++++- Kanal2: MW = 1

Figur 15

# Relatives Füllungsvolumen 1.Art    3 Diabetes,Typ I WHO

Pat.Nr.:3; mess0001.70s; 6.10.1993, 15:56Uhr

relatives Füllungsvolumen **FV**

langwellige Grundperioden

$\Delta FV$

angenäherter Verlauf des mittleren Füllungs- volumens am Meßort Fingerbeere

Pulsnummer n

-*****- Kanal1: MW = 1

Figur 16

Figur 17